(19) Europäisches Patentamt
European Patent Office
Office européen des brevets

(11) **EP 3 207 875 A1**

(12) **EUROPEAN PATENT APPLICATION**

(43) Date of publication:
**23.08.2017 Bulletin 2017/34**

(51) Int Cl.:
*A61B 8/00* (2006.01)   *G01S 15/89* (2006.01)
*A61B 8/08* (2006.01)   *G10K 11/34* (2006.01)

(21) Application number: **16188815.1**

(22) Date of filing: **14.09.2016**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **25.01.2016 KR 20160008512**

(71) Applicants:
• **SAMSUNG MEDISON CO., LTD.**
**Hongcheon-gun, Gangwon-do (KR)**
• **Sogang University Research Foundation**
**Seoul 04107 (KR)**

(72) Inventors:
• LEE, Woo Youl
Seoul (KR)
• SONG, Tae-kyong
Seoul (KR)
• KANG, Hyun Gil
Daejeon (KR)
• PARK, Ji Won
Seoul (KR)

(74) Representative: **Grootscholten, Johannes A.M. et al**
**Arnold & Siedsma**
**Bezuidenhoutseweg 57**
**2594 AC The Hague (NL)**

(54) **BEAMFORMING APPARATUS, ULTRASONIC PROBE HAVING THE SAME, ULTRASONIC DIAGNOSTIC APPARATUS, AND CONTROLLING METHOD THEREOF**

(57)    The present disclosure provides a beamforming apparatus, ultrasonic probe having the same, ultrasonic diagnostic apparatus, and controlling method thereof, which increases the quality of an image in a region of interest by adjusting time delays of analog and digital beamformers in a two dimensional (2D) transducer of the ultrasonic diagnostic apparatus.

In accordance with an aspect of the present disclosure, a beamforming apparatus for beamforming ultrasound received through a two dimensional (2D) arrayed ultrasonic transducer is provided. The apparatus includes an analog beamformer for delaying analog signals received from a subarray including at least one array of the transducer; an analog-to-digital converter (ADC) for converting an analog signal to a digital signal; a digital beamformer for beamforming the digital signal; and a beamformer controller for calculating an initial time delay based on a reference focus point corresponding to a region of interest, and determining a starting point of a sample and hold (S/H) circuit included in the analog beamformer.

**FIG. 10**

START

1001 IS SET REGION OF INTEREST INPUT? — NO

YES

1002 SET RFP IN REGION OF INTEREST

1003 CALCULATE IDEAL TIME DELAY AT RFP

1004 CALCULATE FIRST SYSTEM DELAY FOR RFP

1005 CALCULATE INITIAL TIME DELAY

1006 CALCULATE SECOND SYSTEM DELAY DEPENDING ON DEPTH

1007 CALCULATE DYNAMIC TIME DELAY BASED ON SECND SYSTEM DELAY AND INITIAL TIME DELAY

1008 PERFORMING BEAMFORMING BY APPLYING CALCULATED DELAY

END

**EP 3 207 875 A1**

**Description**

BACKGROUND

## 1. Field of the Invention

The present disclosure relates to a beamforming apparatus, ultrasonic probe having the same, ultrasonic diagnostic apparatus, and controlling method thereof, and more particularly, to a beamforming apparatus, ultrasonic probe having the same, ultrasonic diagnostic apparatus, and controlling method thereof that provides an image optimized in a region of interest.

## 2. Discussion of Related Art

An ultrasonic diagnostic apparatus is used for medical purposes, such as non-invasively acquiring images of layers of soft tissue or blood flows of an internal part of an object by irradiating ultrasound signals generated from transducers of a probe from the surface of the object toward a target part inside the object and receiving information of reflected ultrasound signals (echo ultrasound signals), observing the internal part of the object, detecting foreign materials, analyzing damage, etc.

Compared to other diagnostic imaging apparatuses, such as X-ray diagnostic apparatuses, X-ray Computerized Tomography (CT) scanners, Magnetic Resonance Imaging (MRI) apparatuses, nuclear medicine diagnostic apparatuses, etc., the ultrasonic diagnostic apparatus has many advantages that they are compact, inexpensive, able to display in real time, and safe because of no exposure to radiation, and is thus widely used with other kinds of diagnostic imaging apparatus. A typical ultrasonic diagnostic apparatus provides information about a cross-section of an internal part of a target in two dimensional (2D) images by using a transducer of one dimensional (1D) array. In general, a user (or an examiner, e.g., a doctor) manually or mechanically moves the 1D array transducer (free-hand scan or mechanical scan) to acquire volume information (or three dimensional (3D) information) of an internal part of a target.

However, such a method for acquiring a 3D image through manual or mechanical movement of the 1D array transducer has performance limitations in the aspect of temporal resolution or spatial resolution, so there is a growing interest in a technology to obtain 3D images with a 2D arrayed transducer.

The 2D transducer is required in creating a 3D ultrasound image among images created by the ultrasonic diagnostic apparatus, which makes the hardware that implements beamforming excessively big to support the 2D transducer.

In addition, the use of the 2D arrayed transducer requires more amount of calculations than using the 1D array transducer, thus making the system more complicated. A need exists to develop a technology to prevent an increase in complications of a system and achieve improvement in image resolution and scanning speed.

SUMMARY OF THE INVENTION

The present disclosure provides a beamforming apparatus, ultrasonic probe having the same, ultrasonic diagnostic apparatus, and controlling method thereof, which increases the quality of an image in a region of interest by adjusting time delays of analog and digital beamformers in a two dimensional (2D) transducer of the ultrasonic diagnostic apparatus.

In accordance with an aspect of the present disclosure, a beamforming apparatus for beamforming ultrasound received through a two dimensional (2D) arrayed ultrasonic transducer is provided. The apparatus includes an analog beamformer for delaying analog signals received from a subarray including at least one array of the transducer; an analog-to-digital converter (ADC) for converting an analog signal to a digital signal; a digital beamformer for beamforming the digital signal; and a beamformer controller for calculating an initial time delay based on a reference focus point corresponding to a region of interest, and determining a starting point of a sample and hold (S/H) circuit included in the analog beamformer.

The beamformer controller may determine the starting point by calculating an ideal time delay to be applied for each array from the reference focus point.

The beamformer controller may calculate a first system delay based on the ideal time delay, and calculate the initial time delay based on the first system delay.

The beamformer controller may calculate a second system delay depending on depth.

The beamformer controller may calculate a dynamic time delay based on the second system delay and the initial time delay.

The beamformer controller may control an operation frequency of at least one shift register included in the analog beamformer based on the dynamic time delay.

the beamformer controller may control the digital beamformer based on the first system delay and the second system delay.

In accordance with another aspect of the present disclosure, an ultrasonic diagnostic apparatus is provided. The ultrasonic diagnostic apparatus includes a probe including a two dimensional (2D) arrayed transducer; an image processor for processing an image sent from the probe; a display for displaying an image sent from the image processor and a region of interest; and a controller for controlling the probe and the display, calculating an initial time delay based on a reference focus point corresponding to the region of interest, and determining a starting point of a sample and hold (S/H) circuit included in the analog beamformer, wherein the probe comprises an analog beamformer for delaying analog signals received from a subarray including at least one array of the transducer; an analog-to-digital converter (ADC) for converting an analog signal to a digital signal; a digital beamformer for beamforming the digital signal; and a beamformer controller for controlling at least one of the analog beamformer and the digital beamformer under the control of the controller.

The controller may determine the starting point by calculating an ideal time delay to be applied for each array from the reference focus point.

The controller may calculate a first system delay based on the ideal time delay, and calculate the initial time delay based on the first system delay.

The controller may calculate a second system delay depending on depth.

The controller may calculate a dynamic time delay based on the second system delay and the initial time delay.

The controller may control an operation frequency of at least one shift register included in the analog beamformer based on the dynamic time delay through the beamformer controller.

The controller may control the digital beamformer based on the first system delay and the second system delay through the beamformer controller.

The ultrasonic diagnostic apparatus may further include an input unit for receiving the region of interest set by a user.

## BRIEF DESCRIPTION OF THE DRAWINGS

The above and other objects, features and advantages of the present disclosure will become more apparent to those of ordinary skill in the art by describing in detail exemplary embodiments thereof with reference to the accompanying drawings, in which:

FIG. 1 is a perspective view of an ultrasonic diagnostic apparatus, according to an embodiment of the present disclosure;
FIG. 2 is a control block diagram of an ultrasonic diagnostic apparatus, according to an embodiment of the present disclosure;
FIG. 3 illustrates operation of beamforming by means of one dimensional (1D) array transducer;
FIG. 4; illustrates time delays in received signals to be applied in receive beamforming
FIG. 5 is a detailed control block diagram of a beamforming apparatus;
FIG. 6 is a block diagram of a beamformer, according to an embodiment of the present disclosure;
FIG. 7 is a detailed arrangement of an analog Random Access Memory (RAM) shown in FIG. 6;
FIG. 8 illustrates subarrays in a 2D transducer;
FIG. 9 illustrates time delays of subarrays;
FIG. 10 is a flowchart for providing a sharp image in a region of interest, according to an embodiment of the present disclosure;
FIG. 11 illustrates a 2D transducer in which subarrays and arrays are mathematically distinguished from each other;
FIG. 12 is a diagram for explaining a common equation for calculating a time delay from a focus point to a transducer;
FIG. 13 is a diagram for explaining how to calculate a first system delay from a reference focus point (RFP);
FIG. 14 is a diagram to distinguish a RFP from an arbitrary focus point; and
FIG. 15 is a graph illustrating an effect of an ultrasonic diagnostic apparatus, according to an embodiment of the present disclosure.

## DETAILED DESCRIPTION OF EXEMPLARY EMBODIMENTS

Embodiments of a beamforming apparatus, probe having the same, ultrasonic diagnostic apparatus and controlling method thereof will be described in detail with reference to accompanying drawings. Like reference numerals refer to like components throughout the the drawings, and thus the related descriptions that overlap will be omitted.

The term "object" as herein used may include a person or animal, or a part of the person or animal. For example, the object may include an organ, such as the liver, heart, uterus, brain, breasts, abdomen, etc., or a vein as well as a mass. The term 'user' as herein used may be a doctor, a nurse, a medical technologist, a medical image expert, etc., or a technician who fixes medical equipment, but is not limited thereto.

The terms "ultrasound image" and "object image" as herein used may include an image of an object, which is obtained not only using ultrasounds, but also using an X-ray diagnostic apparatus, a Computerized Tomography (CT) scanner, a Magnetic Resonance Image (MRI) device, or a nuclear medicine diagnostic apparatus.

A diagnostic apparatus for which technologies of an ultrasonic diagnostic apparatus and method for creating an ultrasound image in accordance with embodiments of the present disclosure may be applied or used may be expanded to one of an X-ray scanning apparatus, an X-ray fluoroscopic apparatus, a CT scanner, an MRI, a positron emission tomography apparatus, and an ultrasonic diagnostic apparatus. Embodiments of the present disclosure takes the ultrasonic diagnostic apparatus as an example, without being limited thereto.

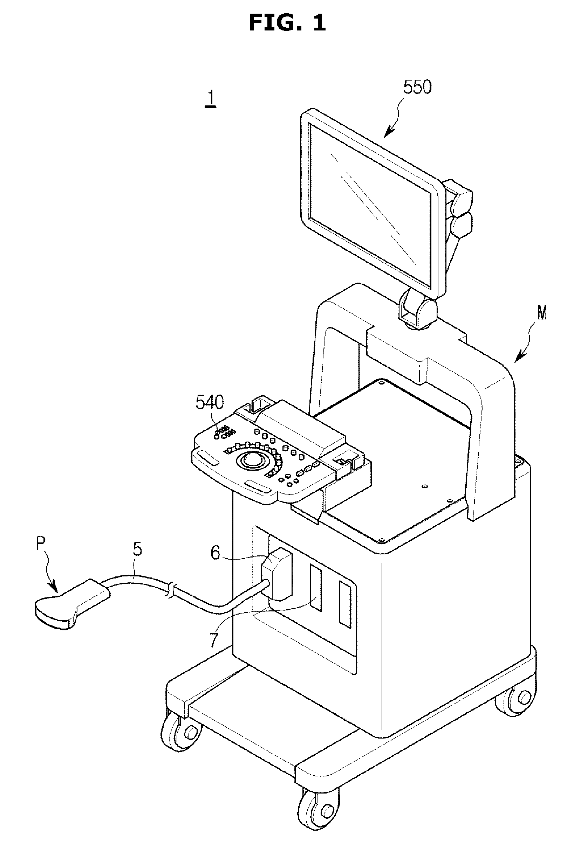

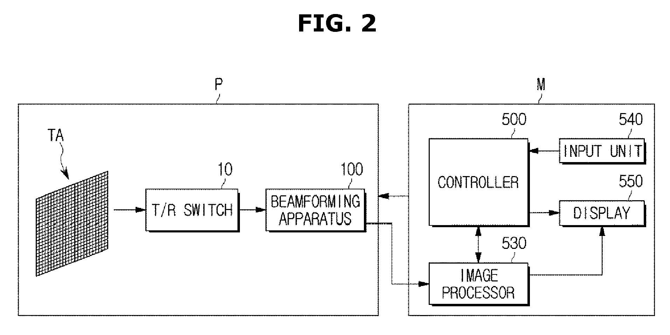

FIG. 1 is a perspective view of an ultrasonic diagnostic apparatus, according to an embodiment of the present disclosure. FIG. 2 is a control block diagram of an ultrasonic apparatus, according to an embodiment of the present disclosure.

Referring to FIG. 1, an ultrasonic diagnostic apparatus 1 may include an ultrasonic probe P that transmits ultrasound to an object, receives echo ultrasound from the object and converts the echo ultrasound to an electric signal, and a main body M connected to the ultrasonic probe P and equipped with an input unit 540 and a display 550 for displaying ultrasound images.

The ultrasonic probe P may be connected to the main body M of the ultrasonic diagnosis apparatus via a cable 5, for receiving various signals required to control the ultrasonic probe P or forwarding analog or digital signals that correspond to ultrasonic echo signals received by the ultrasonic probe P to the main body M.

However, embodiments of the ultrasonic probe P are not limited thereto, and may be implemented with a wireless probe to exchange signals with the main body M over a network formed between the ultrasonic probe P and the main body M.

One end of the cable 5 may be connected to the ultrasonic probe P, and the other end of the cable 5 may be connected to a connector 6 that may be combined with or detachable from a slot 7 of the main body M. The main body M and the ultrasonic probe P may exchange control commands or data via the cable 5.

For example, if the user sets a focal depth or a region of interest on a created ultrasound image through the input unit 540, the information may be forwarded to the ultrasonic probe P via the cable 5 and used by a beamforming apparatus 100.

Alternatively, in the case that the ultrasonic probe P is implemented with a wireless probe as described above, the ultrasonic probe P is connected to the main body M not through the cable 5 but through a wireless network. Even in the case that the ultrasonic probe P is connected to the main body M over a wireless network, control commands or data may be exchanged between the main body M and the ultrasonic probe P.

Referring to FIG. 2, the main body M may include a controller 500, an image processor 530, an input unit 540, and a display 550.

The controller 500 controls general operation of the ultrasonic diagnostic apparatus 1. Specifically, the controller 500 generates control signals to control the respective components of the ultrasonic diagnostic apparatus 1, e.g., a T/R switch 10, the beamforming apparatus 100, the image processor 530, the display 550, etc., as shown in FIG. 2.

Especially, the controller 500 may calculate a delay profile for a plurality of ultrasonic transducer elements (e) that constitute a 2D ultrasonic transducer array (TA), and calculate time delays depending on differences in distance between the plurality of ultrasonic transducer elements (e) included in the 2D ultrasonic transducer array (TA) and a focal point based on the calculated delay profile.

The controller 500 may then control a beamformer 300 of the probe P accordingly to generate transmit/receive signals.

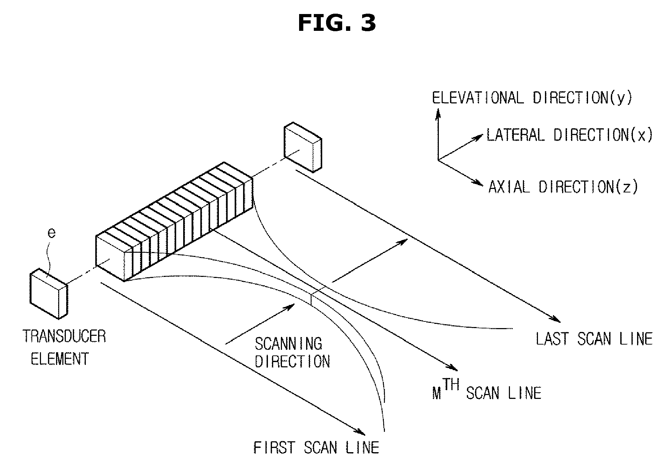

The controller 500 may also generate a sharp image by calculating a time delay at a particular point in a region of interest while performing beamforming on the region of interest. This will be described in detail later in connection with FIG. 3.

Meanwhile, the controller 500 may generate control commands for the respective components of the ultrasonic diagnostic apparatus 1 to control the ultrasonic diagnostic apparatus 1, according to instructions or commands from the user input through the input unit 540.

The controller 500 may also control a beamformer controller 350 of the probe P. That is, the controller 500 may control operation of the beamformer 300 by controlling the beamformer controller 350 through the calculated time delays.

While the controller 500 and the beamformer controller 350 are separately described herein, a process of calculation required for beamforming may be performed by the beamformer controller 500.

The image processor 530 may create an ultrasound image of a target part inside an object based on ultrasound signals focused by the beamforming apparatus 100.

Specifically, the image processor 530 may create a coherent 2D or 3D image of the target part inside the object based on the focused ultrasound signals.

The image processor 530 may also convert the coherent image information to ultrasound image information for a diagnostic mode, such as Brightness mode (B-mode), Doppler mode (or D-mode), etc. For example, if the diagnostic mode is set to the B-mode, the image processor 530 may perform e.g., an analog-to-digital (A/D) conversion process and compose ultrasound image information in real time for an image of the B-mode.

In another example, if a scan mode is set to the D-mode, the image processor 530 may extract phase-change information from an ultrasound signal, calculate information about e.g., blood flow at each point in the scanned cross-section, such as speed, power, or dispersion, and compose ultrasound image information in real time for a D-mode image.

The input unit 540 may allow the user to input a command to operate the ultrasonic diagnostic apparatus 1.

The user may input or set a command to start diagnosis, a command to select a diagnostic mode, such as Amplitude mode (A-mode), B-mode, Color mode (C-mode), D-mode, and Motion mode (M-mode), a location of a region of interest, setting information, etc., through the input unit 540.

The input unit 540 may include various means for the user to input data, instructions, or commands, such as a keyboard, a mouse, a trackball, a tablet, a touch screen module, etc.

The display 550 may display menus or instructions required in ultrasonic diagnosis, and an ultrasound image obtained in the process of the ultrasonic diagnosis. The display 550 may display an ultrasound image of a target part inside an object, which is created by the image processor 530. The ultrasound image to be displayed in the display 550 may be an ultrasound image in the A-mode or B-mode, or may be a 3D ultrasound image. The display 550 may be implemented in various display schemes known to the public, such as Cathode Ray Tube (CRT), Liquid Crystal Display (LCD), etc.

Meanwhile, the ultrasonic diagnostic apparatus 1 may include other components in addition to what are described above, without being limited thereto.

In an embodiment, as shown in FIG. 2, the ultrasonic probe P may include the transducer array TA, the T/R switch 10, and the beamforming apparatus 100.

The transducer array TA is arranged at an end of the ultrasonic probe P. The ultrasonic transducer array TA refers to an array of a plurality of ultrasonic transducer elements e.

In an embodiment, the transducer array TA is in the form of a 2D array.

The ultrasonic transducer array TA generates ultrasound while oscillating due to a pulse signal or alternate current (AC) applied to the ultrasonic transducer array. The ultrasound is transmitted to the target part inside the object. In this case, the ultrasound generated by the ultrasonic transducer array TA may be focused on and transmitted to multiple target parts inside the object. That is, the ultrasound may be multi-focused and transmitted to the multiple target parts.

The ultrasound generated by the ultrasonic transducer array TA may be reflected off at least one target part inside the object and may return to the ultrasonic transducer array TA. The ultrasonic transducer array TA may then receive the echo ultrasound reflected back from the at least one target part.

When the echo ultrasound arrives at the ultrasonic transducer array TA, the ultrasonic transducer array TA may oscillate at a certain frequency corresponding to a frequency of the echo ultrasound and output alternate current at a frequency corresponding to the oscillation frequency of the ultrasonic transducer array TA. Accordingly, the ultrasonic transducer TA may convert the received echo ultrasound to a certain electric signal.

Since each transducer element e receives the echo ultrasound and outputs the electric signal, the ultrasonic transducer array TA may output electric signals on multiple channels. The number of the channels may be the same as the number of ultrasonic transducer elements (e) that constitute the ultrasonic transducer array TA.

However, if the transducer array TA forms a 2D array as in the embodiment of the disclosure, the number of the channels increases drastically compared to when the transducer array TA forms a 1D array. The increased number of channels make the system complicated, increase costs required to implement the system, and make it difficult to implement compact system.

Accordingly, in an embodiment, provided is an ultrasonic diagnostic apparatus 1 that creates a sharp image in a region of interest using the 2D arrayed transducer without increasing the number of channels. This will be described in more detail later.

The ultrasonic transducer elements (e) may include piezoelectric oscillators or thin films. When alternate current is applied to the piezoelectric oscillators or thin films from a power source, the piezoelectric oscillators or thin films oscillate at a certain frequency due to the applied alternate current and generate ultrasound with the certain frequency. On the other hand, the piezoelectric oscillators or thin films oscillate at a certain frequency of an echo ultrasound when the echo ultrasound arrives at the piezoelectric oscillators or thin films, and output alternate current of a frequency corresponding to the oscillation frequency.

For the ultrasonic transducers, e.g., magnetostrictive ultrasonic transducers that use magnetostrictive effect of a magnetic substance, piezoelectric ultrasonic transducers that use piezoelectric effect of a piezoelectric substance, or capacitive Micromachined Ultrasonic Transducers (cMUTs) that transmit and receive ultrasounds by means of oscillation of hundreds or thousands of micromachined thin films may be used.

In addition, other types of transducer that may generate ultrasound from an electrical signal or generate an electrical signal from ultrasound may also be an example of the aforementioned ultrasonic transducer.

The beamforming apparatus 100 may apply transmit pulses for the transducer array TA to transmit an ultrasound signal to a target part inside the object. The beamforming apparatus 100 may also perform a certain process and receive beamforming on the echo ultrasound signal received from the transducer array TA.

A series of processes and beamforming performed by the beamforming apparatus 100 in accordance with an embodiment will now be described in more detail in connection with FIGS. 3 to 5.

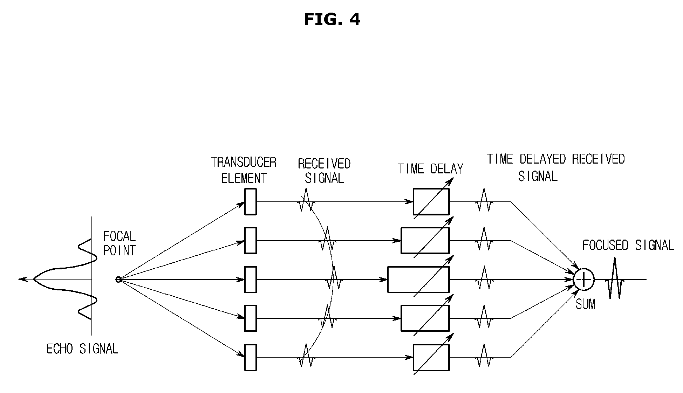

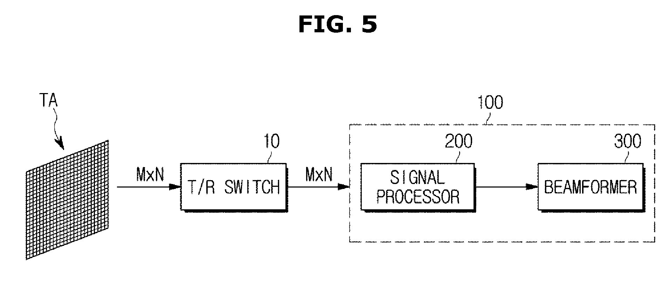

FIG. 3 illustrates operation of beamforming by means of a 1D array transducer, and FIG. 4 illustrates time delays in a received signal to be applied in receive beamforming, and FIG. 5 is a detailed control block diagram of a beamforming apparatus.

Although a 2D transducer array is used in this embodiment, beamforming will be described by taking an example of a 1D transducer array for convenience of explanation.

Referring to FIG. 3, a 3D space subject to ultrasonic imaging may be defined by the x-axis corresponding to a lateral direction, the y-axis corresponding to an elevational direction, and the z-axis corresponding to an axial direction.

Spatial resolution of a 2D ultrasound image may be determined based on axial and lateral resolution. Axial resolution refers to an ability to distinguish two objects that lie along the axis of an ultrasound beam, and the lateral resolution refers to an ability to distinguish two objects that lie in the lateral direction.

Axial resolution is determined by the pulse width of a transmit ultrasound signal, and a higher frequency ultrasound signal with a shorter pulse width yields better axial resolution. Since the lateral resolution and elevational resolution are determined by the width of the ultrasound beam, the narrower the width of the ultrasound beam, the better the lateral resolution.

Accordingly, to improve the resolution of an ultrasound image, in particular, the lateral resolution of the ultrasound image, an ultrasound beam with narrow beamwidth may be formed by focusing ultrasound signals transmitted from a plurality of transducer elements (e) to a transmit focal point on the scan line, which is called transmit beamforming.

The 1D arrayed transducer is comprised of a plurality of transducer elements (e) arrayed in one dimension. To obtain 2D ultrasound cross sectional images, a plurality of scan lines are required and beamforming may be performed for the focal point, as described above, from the first scan line to the last scan line.

A 2D ultrasound cross sectional image on the XY plane may be obtained by transmitting ultrasound signals to all the scan lines and receiving the ultrasound echo signals bouncing back from the internal substances of the object.

To focus ultrasound beams on a spot, the ultrasound signals transmitted from the plurality of transducer elements (e) have to simultaneously arrive at the spot.

However, since the distances from the respective transducer elements (e) to the focal point are different, appropriate time delays are applied to the ultrasound signals to be transmitted from the transducer elements (e) (hereinafter, simply referred to as 'elements') such that the ultrasound signals may arrive at the focal point at the same time.

If the ultrasound signals are transmitted from all the elements (e) to the focal point at the same time, an ultrasound signal transmitted from an element nearest to the focal point arrives first at the focal point and arrival of an element farther from the focal point is delayed.

Accordingly, in applying a transmit signal to the respective elements (e), taking into account the time delay, the transmit signal may be the last to be applied to the element nearest to the focal point while applied earlier to an element farther from the focal point. The transmit signal herein refers to an electric signal applied to the element (e).

Meanwhile, the receive beamforming process is performed backwards from the transmit beamforming process. The ultrasound echo signal reflecting back from the focal point is input to the transducer array (TA), which in turn converts the input ultrasound echo signal to an analog electric signal (hereinafter, simply called electric signal).

Receive beamforming will be described in detail in connection with FIG. 4. As described above in connection with FIG. 3, when ultrasound signals in phase arrive at the focal point by performing transmit beamforming, echo ultrasound signals are produced at the focal point and return to the transducer array TA.

Similar to when the ultrasound signals are to be transmitted to the focal point, distances from the respective transducer elements (e) to the focal point are different when the echo ultrasound is received from the focal point, so the echo ultrasound signals arrive at the respective transducer elements (e) at different points of time.

Specifically, the echo ultrasound signal arrives first at an element nearest to the focal point, and arrives last at an element farthest from the focal point.

Since the magnitude of the echo ultrasound signal is very small, a single echo ultrasound signal received by each element (e) is not enough to obtain necessary information. Therefore, similar to the transmit beamforming process, the receive beamforming process includes applying appropriate time delays to the receive signals arriving at the respective elements (e) at certain intervals and combining the received signals with time delays applied at the same time, thereby improving the signal to noise ratio (SNR).

Referring to FIG. 5, the beamforming apparatus 100 may include a signal processor 200 and a beamformer 300.

An electric signal converted by the transducer array TA may be input to the signal processor 200. The signal processor 200 may amplify the electric signal converted from the echo ultrasound signal prior to performing a signal process or time-delay process, and adjust the gain or compensate for attenuation from the depth.

More specifically, a receive signal processor 220 may include a low noise amplifier (LNA) for reducing noise of the electric signal input from the ultrasonic transducer array TA, and a variable gain amplifier (VGA) for adjusting a gain value based on the input signal. The VGA may correspond to a time gain compensation (TGC) amplifier that compensates for a gain based on a distance to the focal point, without being limited thereto.

The beamformer 300 may perform the aforementioned beamforming on the electric signal received from the signal processor 200. The beamformer 300 may perform signal intensification through superposition of the electric signals received from the signal processor 200.

How the beamformer 300 assigns an appropriate time delay in accordance with an embodiment will now be described in detail.

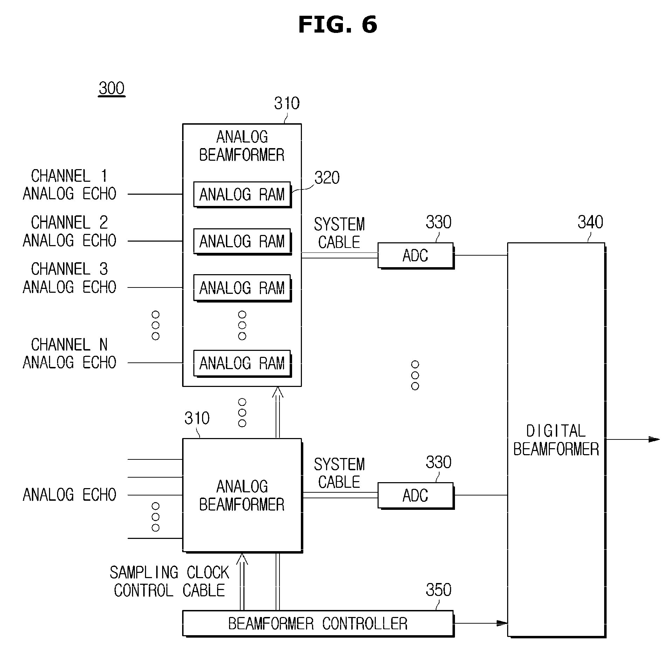

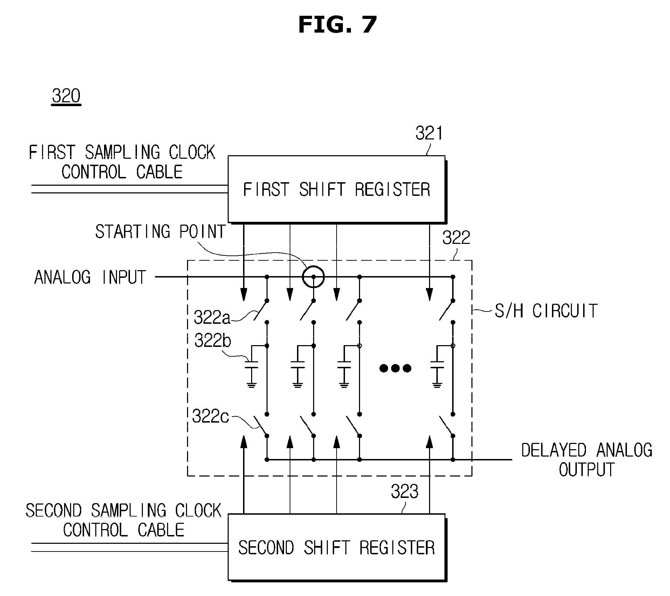

FIG. 6 is a block diagram of a beamformer, according to an embodiment of the present disclosure. FIG. 7 is a detailed arrangement of an analog Random Access Memory (RAM) shown in FIG. 6.

Referring to FIG. 6, the beamformer 300 may include an analog beamformer 310 that has a certain number of analog RAMs 320, an analog-to-digital converter (ADC) 330, a digital beamformer 340, and a beamformer controller 350 for controlling the analog and digital beamformers 310 and 340.

As described above, in the case of beamforming by means of a 2D probe, 1D probes in which transducer elements are arrayed in the horizontal direction are arranged in the vertical direction.

Using the 2D probe in digital beamforming, however, may make the beamformer hardware that supports the 2D transducer too big.

For example, since the digital beamformer 340 performs beamforming or other signal processing only after the analog echo signals in the respective channels are converted to digital signals, each channel requires an ADC. If as many ADCs as the number of the transducer elements (e) are to be installed, the hardware size and complexity used in beamforming may unacceptably increase.

To address this problem, a hybrid beamforming scheme may be used in an embodiment of the present disclosure. Hybrid beamforming means a beamforming scheme that carries out both analog beamforming and digital beamforming. In other words, in the hybrid beamforming, analog beamforming is performed within respective subarrays, and then digitial beamforming is performed for subarrays. Herein, a subarray represents a combination of arrays, when a predetermined number of elements of the transducers are grouped as an array, and a predetermined number of arrays are grouped as a subarray.

As described above in connection with FIG. 4, the analog beamforming is performed by delaying the analog echo signals of the elements (e) or the arrays by different periods of time and then combining values sampled from the elements (e) or the array within the subarray at a particular point of time in an analog method.

The ADC 330 may then convert the analog signal that went through beamforming to a digital signal and forward the digital signal to the digital beamformer 340.

As such, in the hybrid beamforming, analog beamforming is performed within the subarrays and digital beamforming is prformed through the ADC. This may reduce the number of ADCs, and is thus effective in reducing the hardware dimension.

Meanwhile, the analog beamformer 310 may include a plurality of analog RAMs 320. Referring to FIG. 7, in the embodiment of the present disclosure, the analog RAM 320 of FIG. 6 may include two shift registers 321, 323 and a sample/hold (S/H) circuit 322. The analog RAM 320 may also include a charge integrator (not shown).

Specifically, the S/H circuit 322 may include sample switches 322a, sampling capacitors 322b, and read-out switches 322c.

A method implemented in hardware for applying different time delays is achieved by shifting logic '1' (flip-flop method).

In an embodiment, the first shift register 321 may set a single output of the S/H circuit 322, i.e., an output of a D flip-flop at a starting point, to '1' while setting all the remaining outputs to '0'.

When the beamformer controller 350 applies first and second sampling clock (system clock) signals, the logic of each D flip-flop may be shifted to the left or right D flip-flop at each rising edge of the clock.

At this time, a received analog signal (or analog input) is sampled by the sampling capacitors 322b. Specifically, as the logic '1' is shifted in the first shift register 321 that drives the sample switches 322a, sampling is performed at the respective capacitors 322b in sequence.

Read-out operation is also performed at the respective capacitors 322b as the logic '1' is shifted in the second shift register 113 that drives the read-out switches 322c.

As such, the analog RAM 320 makes signals of the respective elements have different time delays by making a difference in hold time between a sampling point in time and a read-out point in time using the S/H circuit 322.

The operation of the beamformer controller 350 applying the first and second sampling clock (system clock) signals is implemented by adjusting the operation frequency of the shift register 321, 323 via a sampling clock control cable.

As shown in FIG. 7, to adjust the operation frequency, as many sampling clock control cables as the number of all the elements (e) are ideally required. This is because the operation frequency of at least one shift register needs to be adjusted to change the analog time delays of the respective analog RAMs 320.

As such, the hybrid beamforming designed to address the hardware complexity requires many cables, e.g., sampling clock control cables in addition to the system cables that connect the data combined at the analog beamformer 310 to the digital beamformer 340.

To overcome this, the hybrid beamforming performs time delaying by dividing the transducer into certain subarrays.

Specifically, since it is very disadvantageous in the hardware aspect to perform time delaying by installing a plurality of sampling clock control cables for the analog RAM 310 for all the elements (e), the number of the sample clock control cables is reduced by using the same sample clock control cables for a reference subarray in all the subarrays.

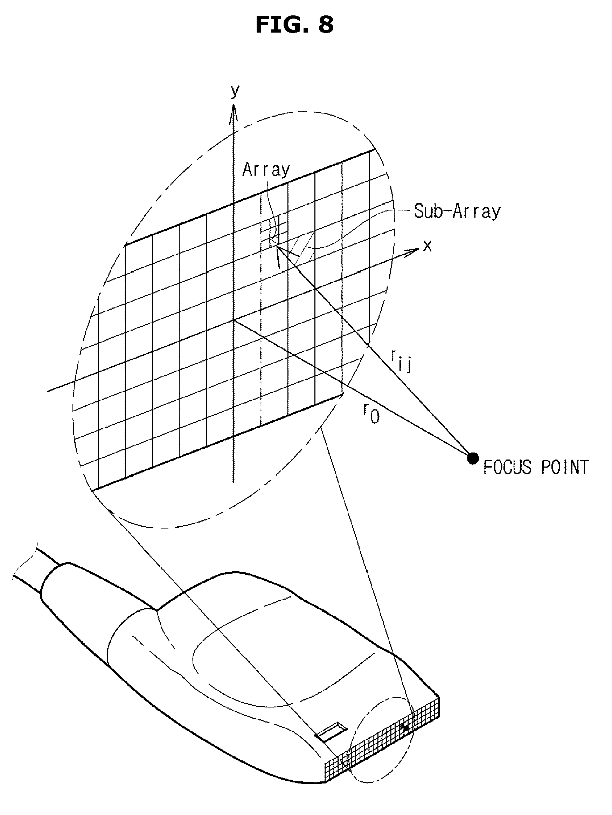

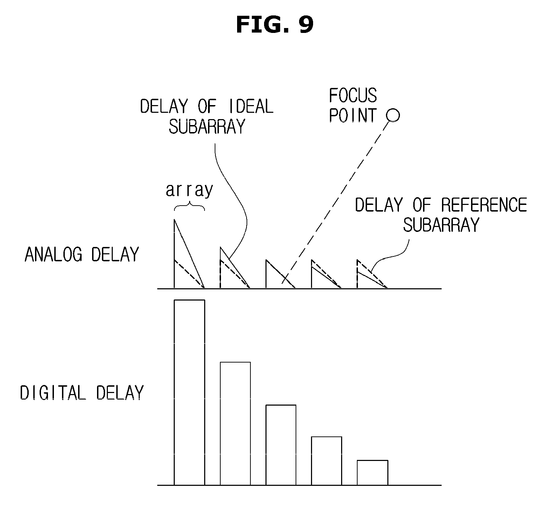

FIG. 8 illustrates subarrays of a 2D transducer, and FIG. 9 illustrates time delays of subarrays. Referring to FIGS. 8 and 9, a problem that causes deterioration of image quality in the beamforming for the divided subarrays will be discussed.

Referring to FIG. 8, the 2D transducer array in accordance with an embodiment of the present disclosure may be comprised of arrays each corresponding to a transducer element (e) and may be divided into a number of subarrays each comprised of a certain number of arrays.

As described above, to reduce the number of the sample clock control cables, the operation frequency is delivered to an analog RAM corresponding to each subarray via a sample clock control cable connected an analog RAM corresponding to a reference subarray.

In other words, it means that the time delay according to a distance between an arbitrary array of each subarray and a focus point is the same as a time delay of an arbitrary array in the reference subarray.

However, since the distance between an array within the subarray and the focus point is different for each subarray, accurate time delays may not be applied. As a result, the time delay applied to the transducers within each subarray in the analog beamforming is the same as that applied to transducers within the reference subarray, which deteriorates quality of the image.

Referring to FIG. 9, values represented by solid lines indicate time delays of an ideal subarray in analog beamforming, and values represented by dotted lines indicate time delays applied to the reference subarray.

If time delays were ideally applied with sample clock control cables connected to the respective analog RAMs 320, different time delays would be applied for the respective distances to the focus point.

However, since the same time delay is applied to the subarrays to reduce complexity of the hardware, the time delay of the reference subarray may be equally applied to each subarray.

Consequently, time delays are not properly reflected in the near depth to the transducer array TA and the region of interest of the object, which prevents clear distinction of an image the object in the region of interest from surrounding images.

To solve this problem, in an embodiment of the present disclosure, initial time delays applied to the respective arrays in the subarray are calculated, thus reducing an error that might occur when a time delay is uniformly applied.

Specifically, the initial time delays determines the position of a starting point as discussed above in connection with FIG. 7. That is, the beamformer controller 350 calculates the initial delay by measuring distances to a reference focus point (RFP), i.e., time delays, and applies different positions of the starting point for the respective arrays, thereby having the same effect of applying time delays via cables.

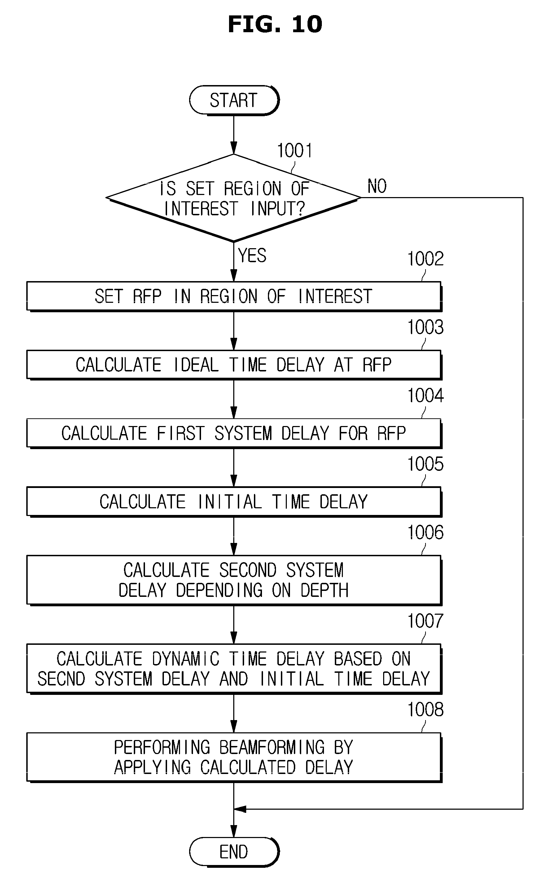

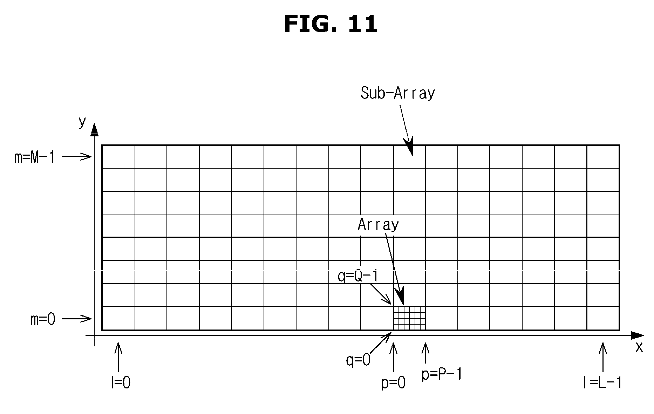

The operation in accordance with an embodiment of the present disclosure will be described in more detail with reference to FIG. 10. FIG. 10 is a flowchart for providing a sharp image in a region of interest, according to an embodiment of the present disclosure. For further description of the respective processes in the flowchart of FIG. 10, reference will be made to FIGS. 11 to 14.

First, the input unit 540 of the ultrasonic diagnostic apparatus 1 determines whether a region of interest is set, in 1001.

Specifically, the image processor 530 creates an ultrasound image based on a signal input from the probe P. The ultrasound image is displayed through the display 550.

The user may set the region of interest on the displayed ultrasound image through the input unit 500, and the controller 500 or the beamformer controller 350 may control the following operations to be performed in relation to the region of interest.

Once the region of interest is set, the controller 500 sets an RFP for the region of interest, in 1002.

The RFP herein refers to a particular focus point at which an accurate time delay may be applied for every scan line for each array of the subarray. In an embodiment of the present disclosure, the initial time delay is calculated based on the RFP to determine a position of the starting point of FIG. 7 for each array of the subarray.

Once the RFP is set, the controller 500 calculates an ideal time delay FSA to be applied for each subarray at the RFP, in 1003.

The ideal time delay FSA is calculated based on the distance between the transducer and the focus point.

FIG. 11 illustrates a 2D transducer, in which subarrays and their arrays are mathematically distinguished.

Referring to FIG. 11, a transducer is comprised of L x M subarrays, each subarray comprises of P x Q transducer array TA. That is, the subarray and the array may be distinguished by (L, M) and (P, Q), respectively.

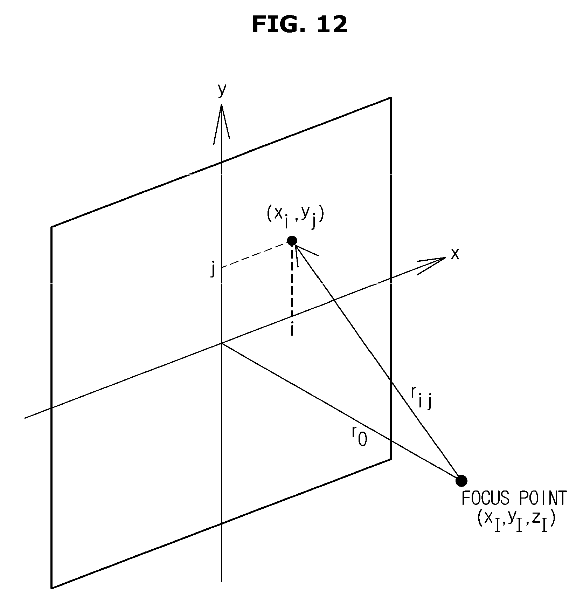

FIG. 12 is a diagram for explaining a common equation for calculating a time delay from a focus point to a transducer.

In FIG. 12, a distance between an arbitrary focus point and a particular transducer array $(x_i, y_j)$ may be calculated in the following equation 1:

$$FSA_{I_{(i,j)}} = r_0 + r_{ij} = \sqrt{x^2 + y^2 + z^2} + \sqrt{(x - x_i)^2 + (y - y_i)^2 + z^2}$$

$$(1)$$

where, $r_O$ represents a distance from the center of the transducer to the focus point, and $r_{ij}$ represents a distance from the focus point to the particular transducer array. That is, a time delay from the focus point to the particular transducer array is represented by a combination of $r_O$ and $r_{ij}$.

Applying this to the ideal time delay FSA results in the following equation 2:

$$FSA_{RF_{(i,j)}} = \sqrt{x^2 + y^2 + z^2} + \sqrt{(x - x_{lP+p})^2 + (y - y_{mQ+q})^2 + z^2}$$

$$(2)$$

where $x_{lP+p}$ and $y_{mQ+q}$ represent the position of each array of the subarray. In other words, the controller 500 may calculate the ideal time delay FSA in equation 2.

Once the ideal time delay FSA is calculated, the controller 500 calculates a first system delay, in 1004.

The term 'system delay' means a time delay applied in the digital beamformer 340 of FIG. 6. Specifically, the system delay refers to a delay equally applied to a plurality of arrays belonging to a particular subarray, and is applied in the process of performing digital beamforming on an output of the analog beamformer 310 stored in the memory.

The first system delay refers to a system delay to be applied at an ideal delay time (FSA).

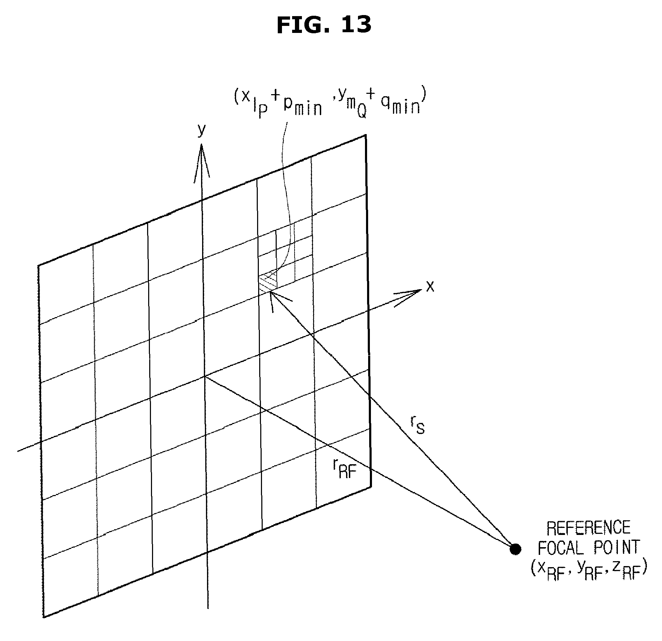

FIG. 13 is a diagram for explaining how to calculate a first system delay from a reference focus point (RFP).

Referring to FIG. 13, the first system delay is calculated based on an array having the smallest delay within the subarray. In the case of the subarray displayed in FIG. 13, an array having the smallest delay is one located in the lower left.

The controller 500 identifies the array having the smallest delay from the subarray to the RFP and calculates the first system delay using the following equation 3:

$$System\ delay_{RF_{(l,m)}} = r_{RF} + r_s$$

$$= \sqrt{x_{RF}^2 + y_{RF}^2 + z_{RF}^2} + \sqrt{(x_{RF} - x_{lP+p_{min}})^2 + (y_{RF} - y_{mQ+q_{min}})^2 + z_{RF}^2}$$

$$(3)$$

where $r_{RF}$ denotes a distance to the $r_{FP}$, and $r_S$ denotes a distance between the RFP and the array having the smallest delay.

The position of the array having the smallest delay is $(x_{lP}+p_{min}, y_{mQ}+q_{min})$.

After the first system delay is calculated, the controller 500 calculates an initial delay, in 1005.

As described above, the initial delay refers to a starting point of the S/H circuit 322 in the analog RAM 320 of FIG. 7. Specifically, when receiving the initial delay calculated by the controller 500, the beamformer controller 350 determines a position of the starting point for each array based on the initial delay.

In this way, the problem that might arise if a time delay were equally applied for each subarray is solved, and an increased quality of image of a region of interest is provided.

The initial delay is calculated using the following equation 4:

$$\text{Initial delay}_{(l_P+p, m_Q+q)} = \text{FSA}_{RF_{(l_P+p, m_Q+q)}} - \text{System delay}_{RF_{(l,m)}}$$

(4)

In other words, the initial delay may be obtained by subtracting the first system delay from the ideal delay (FSA).

After the initial delay is calculated in this way, the controller 500 calculates a second system delay, in 1006.

The second system delay is required to calculate a dynamic delay. The dynamic delay refers herein to a delay that changes with depth.

The dynamic delay is delivered to the shift register of FIG. 7, meaning an operation frequency as described above. That is, the operation frequency delivered by the beamformer controller 350 via the sampling clock control cable is the dynamic delay.

The controller 500 calculates the delay to an arbitrary focus point while maintaining the imaging result for the RFP. For this, the controller 500 obtains the dynamic delay by calculating backwards from the result obtained for the RFP, in 1007.

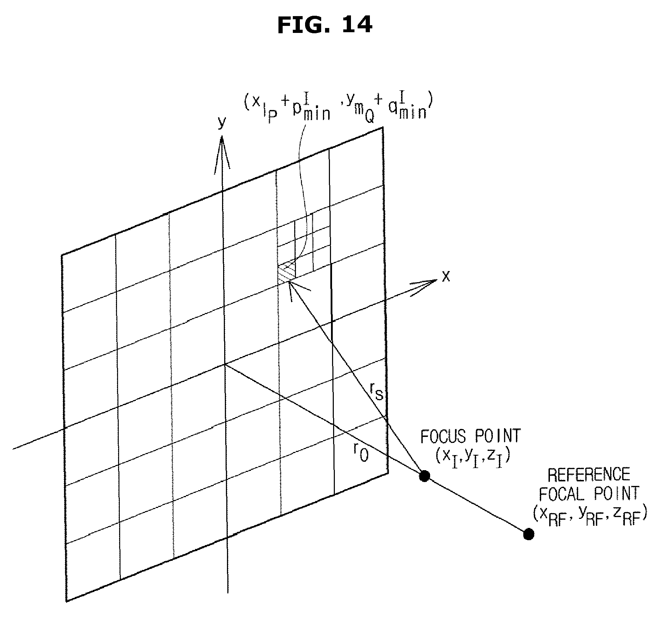

FIG. 14 is a diagram to distinguish an RFP and an arbitrary focus point.

In calculating a delay for an arbitrary focus point as shown in FIG. 14, the controller 500 calculates the second system delay using equation 5. In this case, if the position of a particular array is $\left( x_{lP+p_{min}^I}, \ y_{mQ+q_{min}^I} \right)$ with the arbitrary focus point, the second system delay is calculated as in the following equation 5:

$$\text{System delay}_{I_{(l,m)}} = r_0 + r_s$$
$$= \sqrt{x_I^2 + y_I^2 + z_I^2} + \sqrt{(x_I - x_{lP+p_{min}^I})^2 + (y_I - y_{mQ+q_{min}^I})^2 + z_I^2}$$

(5)

Once the second system delay is calculated in this way, the controller 500 calculates the dynamic delay using equation 6.

As in FIG. 14, assuming that the coordinates of a subarray, which becomes a reference, are (l', m') and the coordinates of a transducer within the subarray is $(x_{l'_P}+p, \ y_{m'_Q}+q)$, the dynamic delay is calculated in the following equation 6.

$$\text{Dynamic Delay}_{(l_P+p, m_Q+q)}$$
$$= \text{FSA}_{RF_{(l'_P+p, m'_Q+q)}} - \text{System delay}_{I_{(l',m')}} - \text{Initial delay}_{(l'_P+p, m'_Q+q)}$$

(6)

Once the dynamic delay is calculated, the controller 500 performs beamforming based on the calculated system delay, initial delay, and dynamic delay, in 1008. As such, the ultrasonic diagnostic apparatus 1 in an embodiment of the present disclosure may display an improved image at the RFP, i.e., in a region of interest.

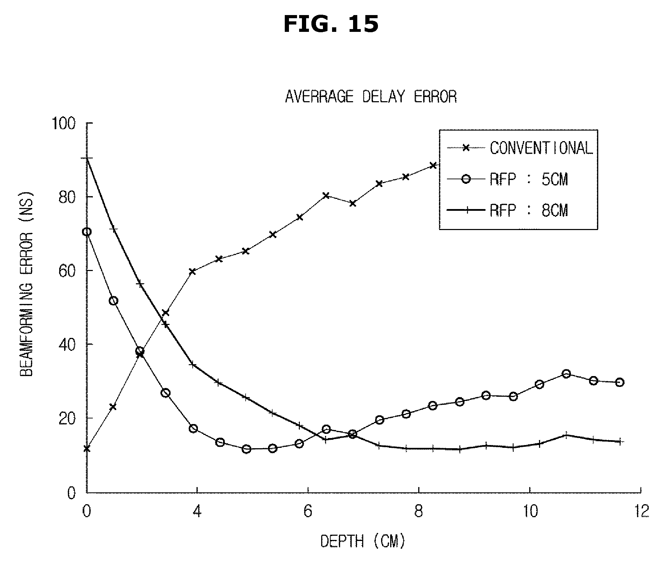

FIG. 15 is a graph illustrating an effect of an ultrasonic diagnostic apparatus, according to an embodiment of the present disclosure.

In FIG. 15, the x-axis represents the depth of an object, i.e., the depth of focal points. The y-axis represents errors produced while beamforming is performed, i.e., beamforming errors.

Referring to the conventional line of FIG. 15, it is seen that a beamforming error increases with the distance from the RFP.

However, according to the present disclosure, it is seen that when a user sets a region of interest to a position of 5cm or 8cm from the RFP, a beamforming error corresponding to the set range decreases.

Specifically, in the case that the RFP is 5cm, the average error decreases from 65.19ns to 12.08ns, and thus it is seen that there is 81% of performance improvement as compared with the conventional technology. Accordingly, the ultrasonic diagnostic apparatus 1 in accordance with embodiments of the present disclosure may provide very sharp images for the region of interest.

According to embodiments of the present disclosure, the quality of an image in a region of interest may be improved by adjusting time delays of analog and digital beamformers in the 2D transducer of the ultrasonic diagnostic apparatus.

**Claims**

1. A beamforming apparatus for beamforming ultrasound received through a two dimensional (2D) arrayed ultrasonic transducer, the beamforming apparatus comprising:

   an analog beamformer for delaying analog signals received from a subarray including at least one array of the transducer;
   an analog-to-digital converter (ADC) for converting an analog signal to a digital signal;
   a digital beamformer for beamforming the digital signal; and
   a beamformer controller for calculating an initial time delay based on a reference focus point corresponding to a region of interest, and determining a starting point of a sample and hold (S/H) circuit included in the analog beamformer.

2. The beamforming apparatus of claim 1, wherein the beamformer controller is configured to determine the starting point by calculating an ideal time delay to be applied for each array from the reference focus point.

3. The beamforming apparatus of claim 2, wherein the beamformer controller is configured to calculate a first system delay based on the ideal time delay, and calculate the initial time delay based on the first system delay.

4. The beamforming apparatus of claim 3, wherein the beamformer controller is configured to calculate a second system delay depending on depth.

5. The beamforming apparatus of claim 4, wherein the beamformer controller is configured to calculate a dynamic time delay based on the second system delay and the initial time delay.

6. The beamforming apparatus of claim 5, wherein the beamformer controller is configured to control an operation frequency of at least one shift register included in the analog beamformer based on the dynamic time delay.

7. The beamforming apparatus of claim 4, wherein the beamformer controller is configured to control the digital beamformer based on the first system delay and the second system delay.

8. An ultrasonic diagnostic apparatus comprising:

   an input unit configured to receive a command related to a transmit focal point;
   a probe including a two dimensional (2D) arrayed transducer;
   an image processor for processing an image sent from the probe;
   a display for displaying an image sent from the image processor and a region of interest; and
   a controller for controlling the probe and the display, calculating an initial time delay based on a reference focus point corresponding to the region of interest, and determining a starting point of a sample and hold (S/H) circuit included in the analog beamformer,
   wherein the probe comprises
   an analog beamformer for delaying analog signals received from a subarray including at least one array of the transducer;

an analog-to-digital converter (ADC) for converting an analog signal to a digital signal;
a digital beamformer for beamforming the digital signal; and
a beamformer controller for controlling at least one of the analog beamformer and the digital beamformer under the control of the controller.

9.  The ultrasonic diagnostic apparatus of claim 8, wherein the controller is configured to determine the starting point by calculating an ideal time delay to be applied for each array from the reference focus point.

10. The ultrasonic diagnostic apparatus of claim 9, wherein the controller is configured to calculate a first system delay based on the ideal time delay, and calculate the initial time delay based on the first system delay.

11. The ultrasonic diagnostic apparatus of claim 10, wherein the controller is configured to calculate a second system delay depending on depth.

12. The ultrasonic diagnostic apparatus of claim 11, wherein the controller is configured to calculate a dynamic time delay based on the second system delay and the initial time delay.

13. The ultrasonic diagnostic apparatus of claim 12, wherein the controller is configured to control an operation frequency of at least one shift register included in the analog beamformer based on the dynamic time delay through the beamformer controller.

14. The ultrasonic diagnostic apparatus of claim 11, wherein the controller is configured to control the digital beamformer based on the first system delay and the second system delay through the beamformer controller.

15. The ultrasonic diagnostic apparatus of claim 8, wherein the input unit is configured to receive the region of interest set by a user.

**FIG. 1**

# FIG. 2

# FIG. 3

ELEVATIONAL DIRECTION(y)

LATERAL DIRECTION(x)

AXIAL DIRECTION(z)

e

TRANSDUCER
ELEMENT

SCANNING
DIRECTION

LAST SCAN LINE

$M^{TH}$ SCAN LINE

FIRST SCAN LINE

**FIG. 4**

# FIG. 5

# FIG. 6

**FIG. 7**

320

FIRST SAMPLING CLOCK
CONTROL CABLE

321

FIRST SHIFT REGISTER

STARTING POINT

322

ANALOG INPUT

322a

322b

S/H CIRCUIT

●●●

322c

DELAYED ANALOG
OUTPUT

323

SECOND SAMPLING CLOCK
CONTROL CABLE

SECOND SHIFT REGISTER

# FIG. 8

# FIG. 9

DELAY OF IDEAL SUBARRAY

FOCUS POINT

array

DELAY OF REFERENCE SUBARRAY

ANALOG DELAY

DIGITAL DELAY

# FIG. 10

```
                    ( START )
                        │
                        ▼              1001
                   ╱─────────────╲        NO
              ╱ IS SET REGION OF  ╲──────────────┐
              ╲  INTEREST INPUT?  ╱               │
                   ╲─────────────╱                │
                        │ YES                     │
                        ▼              1002       │
        ┌───────────────────────────────────┐    │
        │   SET RFP IN REGION OF INTEREST    │    │
        └───────────────────────────────────┘    │
                        │              1003       │
                        ▼                         │
        ┌───────────────────────────────────┐    │
        │  CALCULATE IDEAL TIME DELAY AT RFP │    │
        └───────────────────────────────────┘    │
                        │              1004       │
                        ▼                         │
        ┌───────────────────────────────────┐    │
        │  CALCULATE FIRST SYSTEM DELAY FOR RFP │ │
        └───────────────────────────────────┘    │
                        │              1005       │
                        ▼                         │
        ┌───────────────────────────────────┐    │
        │     CALCULATE INITIAL TIME DELAY   │    │
        └───────────────────────────────────┘    │
                        │              1006       │
                        ▼                         │
        ┌───────────────────────────────────┐    │
        │      CALCULATE SECOND SYSTEM       │    │
        │     DELAY DEPENDING ON DEPTH       │    │
        └───────────────────────────────────┘    │
                        │              1007       │
                        ▼                         │
        ┌───────────────────────────────────┐    │
        │ CALCULATE DYNAMIC TIME DELAY BASED ON │ │
        │ SECND SYSTEM DELAY AND INITIAL TIME DELAY │ │
        └───────────────────────────────────┘    │
                        │              1008       │
                        ▼                         │
        ┌───────────────────────────────────┐    │
        │      PERFORMING BEAMFORMING BY     │    │
        │      APPLYING CALCULATED DELAY     │    │
        └───────────────────────────────────┘    │
                        │                         │
                        ▼◄────────────────────────┘
                    ( END )
```

# FIG. 11

# FIG. 12

# FIG. 13

$(x_{l_P} + p_{min}, y_{m_Q} + q_{min})$

y

x

$r_S$

$r_{RF}$

REFERENCE
FOCAL POINT
$(x_{RF}, y_{RF}, z_{RF})$

25

# FIG. 14

# FIG. 15

AVERRAGE DELAY ERROR

Europäisches
Patentamt

European
Patent Office

Office européen
des brevets

# EUROPEAN SEARCH REPORT

Application Number

EP 16 18 8815

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2005/131299 A1 (ROBINSON BRENT [US] ET AL) 16 June 2005 (2005-06-16) * paragraphs [0009] - [0012], [0017], [0018], [0023], [0044] - [0045], [0059] - [0060]; figures 1,5c,6 * ----- | 1-15 | INV. A61B8/00 G01S15/89 A61B8/08 G10K11/34 |
| X | CHEN CHAO ET AL: "A Prototype PZT Matrix Transducer With Low-Power Integrated Receive ASIC for 3-D Transesophageal Echocardiography", IEEE TRANSACTIONS ON ULTRASONICS, FERROELECTRICS AND FREQUENCY CONTROL, IEEE, US, vol. 63, no. 1, 1 January 2016 (2016-01-01), pages 47-59, XP011596523, ISSN: 0885-3010, DOI: 10.1109/TUFFC.2015.2496580 [retrieved on 2015-12-30] * abstract; figures 1,2,4,5 * * page 1, right-hand column, paragraph 3 * * page 2, right-hand column, paragraph 4 - page 3, right-hand column, paragraph 1 * * page 4, right-hand column, paragraph 4 - page 5, right-hand column, paragraph 1 * * page 8, left-hand column, paragraph 2 - right-hand column, paragraph 2 * ----- | 1-15 | |
| A | US 2015/099977 A1 (KIM BAE HYUNG [KR] ET AL) 9 April 2015 (2015-04-09) * paragraphs [0090] - [0112], [0126] - [0137]; figures 9,11 * ----- | 1-15 | TECHNICAL FIELDS SEARCHED (IPC)  A61B G01S G10K G11C |

-/--

The present search report has been drawn up for all claims

| Place of search | Date of completion of the search | Examiner |
|---|---|---|
| Munich | 14 June 2017 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 16 18 8815

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2012/179043 A1 (KIM BAE-HYUNG [KR] ET AL) 12 July 2012 (2012-07-12) * paragraphs [0041] - [0043], [0048], [0057] - [0059], [0062] - [0066], [0071] - [0083], [0098] - [0102], [0109] - [0114]; figures 1-5b,7,9 * ----- | 1-3,8-10 | |
| A | US 2010/228130 A1 (CHIANG ALICE M [US] ET AL) 9 September 2010 (2010-09-09) * paragraphs [0024], [0030] - [0031], [0054], [0118] - [0125], [0181]; figures 25,3-4,6 * ----- | 1-3,8-10 | |
| A | UM JI-YONG ET AL: "A Single-Chip 32-Channel Analog Beamformer With 4-ns Delay Resolution and 768-ns Maximum Delay Range for Ultrasound Medical Imaging With a Linear Array Transducer", IEEE TRANSACTIONS ON BIOMEDICAL CIRCUITS AND SYSTEMS, IEEE, US, vol. 9, no. 1, 1 February 2015 (2015-02-01), pages 138-151, XP011571114, ISSN: 1932-4545, DOI: 10.1109/TBCAS.2014.2325851 [retrieved on 2015-01-23] * abstract; figures 1b,2,3,6 * ----- | 1-15 | TECHNICAL FIELDS SEARCHED (IPC) |

-/--

The present search report has been drawn up for all claims

| Place of search | Date of completion of the search | Examiner |
|---|---|---|
| Munich | 14 June 2017 | Daoukou, Eleni |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

## EUROPEAN SEARCH REPORT

Application Number

EP 16 18 8815

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JAE-HWAN KIM ET AL: "Time-interleaved sample clock generator for ultrasound beamformer application", SOC DESIGN CONFERENCE (ISOCC), 2011 INTERNATIONAL, IEEE, 17 November 2011 (2011-11-17), pages 290-293, XP032101698, DOI: 10.1109/ISOCC.2011.6138767 ISBN: 978-1-4577-0709-4 * page 291, right-hand column, paragraph 2 - page 292, left-hand column, paragraph 3 * ----- | 1-3,8-10 | |
| A | US 2016/011305 A1 (KOPTENKO SERGEI V [CA]) 14 January 2016 (2016-01-14) * paragraphs [0007], [0016] - [0017], [0038], [0058] - [0061], [0077] - [0078]; claim 1; figures 3-6,8b-9,12-13 * ----- | 1,8 | |

TECHNICAL FIELDS
SEARCHED        (IPC)

The present search report has been drawn up for all claims

| Place of search | Date of completion of the search | Examiner |
|---|---|---|
| Munich | 14 June 2017 | Daoukou, Eleni |

EPO FORM 1503 03.82 (P04C01)

## ANNEX TO THE EUROPEAN SEARCH REPORT
## ON EUROPEAN PATENT APPLICATION NO.

EP 16 18 8815

This annex lists the patent family members relating to the patent documents cited in the above-mentioned European search report.
The members are as contained in the European Patent Office EDP file on
The European Patent Office is in no way liable for these particulars which are merely given for the purpose of information.

14-06-2017

| Patent document cited in search report | Publication date | Patent family member(s) | Publication date |
|---|---|---|---|
| US 2005131299 A1 | 16-06-2005 | NONE | |
| US 2015099977 A1 | 09-04-2015 | CN 104510501 A<br>EP 2860547 A1<br>JP 2015073902 A<br>KR 20150041471 A<br>US 2015099977 A1 | 15-04-2015<br>15-04-2015<br>20-04-2015<br>16-04-2015<br>09-04-2015 |
| US 2012179043 A1 | 12-07-2012 | CN 102579078 A<br>EP 2474835 A2<br>JP 2012143559 A<br>KR 20120080093 A<br>US 2012179043 A1 | 18-07-2012<br>11-07-2012<br>02-08-2012<br>16-07-2012<br>12-07-2012 |
| US 2010228130 A1 | 09-09-2010 | NONE | |
| US 2016011305 A1 | 14-01-2016 | US 2016011305 A1<br>WO 2014125371 A1 | 14-01-2016<br>21-08-2014 |

EPO FORM P0459

For more details about this annex : see Official Journal of the European Patent Office, No. 12/82